# EUROPEAN PATENT SPECIFICATION

(11) **EP 3 213 748 B1**
(45) Date of publication and mention of the grant of the patent: **18.08.2021**
(21) Application number: 16201983.0
(22) Date of filing: 04.06.2009
(51) Int. Cl.: A61K 31/138, A61K 31/17, A61K 31/282, A61K 31/352, A61K 31/4535, A61K 31/53, A61K 31/185, A61K 45/06, A61P 25/04, A61P 43/00

(54) **CANNABINOIDS (THC:CBD RATIO) IN COMBINATION WITH AN ALKYLATING AGENT FOR USE IN TREATING BRAIN TUMOUR**
CANNABINOIDE (THC:CBD RATIO) IN KOMBINATION MIT EINEM ALKYLIERENDEN MITTEL ZUR VERWENDUNG IN DER BEHANDLUNG VON GEHIRNTUMOR
CANNABINOÏDES ASSOCIÉS À UN AGENT D'ALKYLATION POUR L'UTILISATION DANS LE TRAITEMENT DU CANCER DU CERVEAU

(30) Priority: 04.06.2008 GB 0810203
(43) Date of publication of application: 06.09.2017
(62) Divisional of application: 09757810.8
(73) Proprietor: GW Pharma Limited, Histon Cambridge CB24 9BZ (GB)
(72) Inventor: VELASCO DIEZ, Guillermo, E-28040 Madrid (ES); GUZMAN PASTOR, Manuel, E-28040 Madrid (ES); LORENTE, Mar, E-28040 Madrid (ES); TORRES, Sofia, E-28040 Madrid (ES)
(74) Representative: HGF

(56) References cited:
- WO-A-2006/107903
- WO-A1-02/069993
- US-A1- 2004 039 048
- JACOBSSON STIG O P ET AL: "Serum-dependent effects of tamoxifen and cannabinoids upon C6 glioma cell viability", BIOCHEMICAL PHARMACOLOGY, vol. 60, no. 12, 15 December 2000 (2000-12-15), pages 1807-1813, XP009121672, ISSN: 0006-2952
- MASSI PAOLA ET AL: "Antitumor effects of cannabidiol, a nonpsychoactive cannabinoid, on human glioma cell lines.", JOURNAL OF PHARMACOLOGY AND EXPERIMENTAL THERAPEUTICS, vol. 308, no. 3, March 2004 (2004-03), pages 838-845, XP002542257, ISSN: 0022-3565
- VELASCO GUILLERMO ET AL: "Cannabinoids and gliomas", MOLECULAR NEUROBIOLOGY, HUMANA PRESS, US, vol. 36, no. 1, 1 August 2007 (2007-08-01) , pages 60-67, XP002540822, ISSN: 0893-7648, DOI: 10.1007/S12035-007-0002-5 [retrieved on 2007-06-28]
- VACCANI A ET AL: "Cannabidiol inhibits human glioma cell migration through a cannabinoid receptor-independent mechanism", BRITISH JOURNAL OF PHARMACOLOGY, WILEY-BLACKWELL, UK, vol. 144, no. 8, 1 April 2005 (2005-04-01) , pages 1032-1036, XP002540821, ISSN: 0007-1188, DOI: 10.1038/SJ.BJP.0706134
- KRAJCI D ET AL: "Ultrastructure of nuclei of cisplatin-treated C6 glioma cells undergoing apoptosis", EUROPEAN JOURNAL OF CELL BIO, WISSENSCHAFLICHE VERLAGSGESELLSCHAFT, STUTTGART, DE, vol. 79, no. 5, 1 May 2000 (2000-05-01), pages 365-376, XP004954373, ISSN: 0171-9335, DOI: 10.1078/S0171-9335(04)70041-2
- ARRIETA O ET AL: "Protamine inhibits angiogenesis and growth of C6 rat glioma; a synergistic effect when combined with carmustine", EUROPEAN JOURNAL OF CA, ELSEVIER, AMSTERDAM, NL, vol. 34, no. 13, 1 December 1998 (1998-12-01), pages 2101-2106, XP004285708, ISSN: 0959-8049, DOI: 10.1016/S0959-8049(98)00244-5
- BERROCAL A ET AL: "Temozolamide in previously treated high-grade gliomas patients", EUROPEAN JOURNAL OF CA, ELSEVIER, AMSTERDAM, NL, vol. 37, 1 April 2001 (2001-04-01), page S343, XP004478514, ISSN: 0959-8049, DOI: 10.1016/S0959-8049(01)81765-2

## Description

The present invention relates to a combination of the cannabinoids tetrahydrocannabinol (THC) and cannabidiol (CBD) in a ratio of from 5:1 to 1:5 together with an alkylating agent for use in the treatment of a brain tumour, wherein the THC and / or CBD is present in an amount which would be considered sub-optimal if it were used alone.

### BACKGROUND TO THE INVENTION

Cancer a disease in which a group of cells display the traits of uncontrolled growth. This means that the cells grow and divide beyond the levels of normal limits. The cells are also able to invade and destroy surrounding tissues. In addition cancer cells sometimes also metastasize, meaning that they spread to other locations in the body via the blood or lymph.

Most cancers are caused by abnormalities in the genetic material of the cells. These abnormalities may be due to the effects of carcinogens. Other cancer-promoting genetic abnormalities may be randomly acquired through errors in DNA replication, or are inherited, and thus present in all cells from birth.

Genetic abnormalities found in cancer typically affect two general classes of genes. Cancer-promoting oncogenes are often activated in cancer cells, giving those cells new properties, such as hyperactive growth and division, protection against programmed cell death, loss of respect for normal tissue boundaries, and the ability to become established in diverse tissue environments.

Tumour suppressor genes are often inactivated in cancer cells, resulting in the loss of normal functions in those cells, such as accurate DNA replication, control over the cell cycle, orientation and adhesion within tissues, and interaction with protective cells of the immune system.

There are many different types of cancer and the cancer is usually classified according to the type of tissue from which it originated.

Cancer is usually treated by one or more of the following: surgery, chemotherapy, radiation therapy, immunotherapy and monoclonal antibody therapy. The type of therapy depends upon the location and grade of the tumour and the stage of the disease.

Complete removal of the cancer without damage to the rest of the body is the goal of treatment. Sometimes this can be accomplished by surgery, but the propensity of cancers to invade adjacent tissue or to spread to distant sites by microscopic metastasis often limits its effectiveness. The effectiveness of chemotherapy is often limited by toxicity to other tissues in the body. Radiation can also cause damage to normal tissue.

Cancers are known to affect many areas of the body with the most common types of cancers including: cancer of the bile duct, cancer of the bladder, cancer of the bone, cancer of the bowel (including cancer of the colon and cancer of the rectum), cancer of the brain, cancer of the breast, cancer of the neuroendocrine system (commonly known as a carcinoid), cancer of the cervix, cancer of the eye, cancer of the oesophagus, cancer of the head and neck (this group includes carcinomas that start in the cells that form the lining of the mouth, nose, throat, ear or the surface layer covering the tongue), Kaposi's sarcoma, cancer of the kidney, cancer of the larynx, leukaemia, cancer of the liver, cancer of the lung, cancer of the lymph nodes, Hodgkin's lymphoma, non-Hodgkin's lymphoma, melanoma, mesothelioma, myeloma, cancer of the ovary, cancer of the pancreas, cancer of the penis, cancer of the prostate, skin cancer, soft tissue sarcomas, cancer of the spinal cord, cancer of the stomach, testicular cancer, cancer of the thyroid, cancer of the vagina, cancer of the vulva and cancer of the uterus.

A tumour that develops in the brain can destroy or damage brain cells by producing inflammation, compressing other parts of the brain, inducing cerebral oedema (brain swelling) and can cause increases in intracranial pressure (pressure within the skull).

Each year, approximately 4300 people in the UK are diagnosed with a brain tumour. A primary brain tumour is a mass created by the growth or uncontrolled proliferation of cells in the brain. Malignant primary brain tumours are most likely to cause problems by spreading into the normal brain tissue which surrounds them and causing pressure and damage to the surrounding areas of the brain. These tumours rarely spread outside the brain to other parts of the body. However, secondary brain tumours occur when cancer cells from other parts of the body, such as the lung or breast spread to the brain.

Surgery is the treatment option of choice for many brain tumours. Some may be completely excised, but those that are deep or that infiltrate brain tissue may be debulked rather than removed.

Radiation therapy and chemotherapy may be recommended depending on the type of tumour involved.

Glioma cell tumours can often be lethal. The characteristic diffuse infiltrative tumour growth of gliomas often makes the surgical removal of them impossible and this profoundly complicates the clinical management of these patients.

Glioblastoma multiforme (GBM) is the most common and most aggressive type of primary brain tumour and accounts for 52% of all primary brain tumour cases and 20% of all intracranial tumours.

Different approaches are being researched in order to improve the mortality rate of patients diagnosed with a glioma. These include therapies that target the glioma cells but leave normal cells unharmed, methods that limit the spread of the cancer cells and treatments that block the tumours life-sustaining molecules.

One such area of research involves the use of cannabinoids as anti-tumoural agents.

Cannabinoids are the active constituents of cannabis plants and they have been found to demonstrate numerous pharmacological properties.

For example EP1177790 (Guzman et al.) describes the treatment of cerebral tumours by the administration of a natural or synthetic cannabinoid, specifically THC. It is claimed that activation of specific receptors leads to selective death of the transformed cells.

Recently the cannabinoid CBD has been shown to possess anti-tumoural properties (Massi et al. 2004). The work described by this paper describes anti-proliferative effects both *in-vitro* using U87 and U373 human glioma cell lines and *in-vivo* using U87 human glioma cells subcutaneously implanted to nude mice.

Biochemical Pharmacology 2000, vol 6, p1807-1813 compared the effects of cannabinoids including THC and CBD with the estrogen receptor modulator Tamoxifen and concluded that the effects of the cannabinoids were modest and that there was no significant interaction between them and Tamoxifen.

Malignant gliomas are highly infiltrative and proliferative tumours, which follow a characteristic pattern of growth. Glioma cells invade the adjacent normal brain structures and surrounding large blood vessels.

In addition the applicant's earlier patent EP1802274 describes the use of the cannabinoid CBD to impede the progress of cancer cells migrating from their primary tumour location to a secondary site.

The applicant's co-pending application GB0810195.8, filed on 4 June 2008, describes the use of a combination of cannabinoids in the treatment of cancer.

### SUMMARY OF INVENTION

According to the present invention there is provided a combination of the cannabinoids tetrahydrocannabinol (THC) and cannabidiol (CBD) in a ratio of from 5:1 to 1:5 together with an alkylating agent for use in the treatment of a brain tumour wherein the THC and/ or CBD is present in an amount which would be considered sub-optimal if it were used alone.

Preferably the THC is present in an amount which would be considered sub-optimal if it were used alone Alternatively the CBD is present in an amount which would be considered sub-optimal if it were used alone. In a further embodiment the THC and CBD are present in an amount which would be considered sub-optimal if they were used alone.

Preferably the treatment of the brain tumour is to reduce cell viability, inhibit cell growth or reduce tumour volume.

More preferably still, the THC and CBD are in the ratio of from between 2:1 to 1:2, more approximately 1:1.

Each cannabinoid is provided in a therapeutically effect amount. Dose ranges for the THC and CBD may be determined by reference to the cannabinoid content which is preferably in the range of between 5 and 100mg of the total cannabinoids.

An alkylating agent is a type of anti-neoplastic agent that works by interfering with DNA in a number of ways.

Extra molecules, called alkyl groups, are added to DNA, which causes it to break apart as the cell tries to replace them. Alkylating agents also interfere with the bonds between DNA strands, stopping them from separating, which is a step required in DNA replication. By replacing bases (important components of DNA) alkylating agents also create mismatching, another way to stop DNA being reproduced properly.

All these changes occur when a cell is preparing to divide, and the permanent damage they cause results in cessation of division and cell death.

Preferably the alkylating agent is selected from the group consisting of: alkyl sulfonates; busulfan; ethyleneimines and methylmelamines; hexamethymelamine; altretamine; thiotepa; nitrogen mustards; cyclophosphamide; mechlorethamine; mustine; uramustine; uracil mustard; melphalan; chlorambucil; ifosfamide; nitrosureas; carmustine; cisplatin; streptozocin; triazenes; decarbazine; imidazotetrazines; and temozolomide.

Alkylating agents used as anti-tumoural agents include: cisplatin, temozolamide and carmustine and these are preferred in the practice of the present invention.

Brain tumours are usually classified according to the location of the tumour and the type of cell that the cancer has developed from.

For example different types of brain tumour include: acoustic neuroma, astrocytoma, CNS lymphoma, ependymoma, haemangioblastoma, medulloblastoma, meningioma, glioma, mixed glioma, oligodendroglioma, pineal region tumours and pituitary tumours.

Gliomas are tumours of the glial cells; these cells support and protect nerve cells in the brain. Gliomas comprise nearly half of all primary brain tumours and a fifth of all primary spinal cord tumours.

The combined therapy of the invention is particularly useful where the brain tumour is a glioma tumour, more particularly glioblastoma multiforme (GBM).

The one or more cannabinoids may be present as plant extracts, as pure compounds, or a combination of the two.

A plant extract is defined as an extract from a plant material as described by the Guidance for Industry Botanical Drug Products Draft Guidance, August 2000, US Department of Health and Human Services, Food and Drug Administration Centre for Drug Evaluation and Research.

Plant material is defined as a plant or plant part (e.g. bark, wood, leaves, stems, roots, flowers, fruits, seeds, berries or parts thereof) as well as exudates.

More preferably the plant extract is in the form of a botanical drug substance.

A botanical drug substance is defined as follows. Botanical drug substances which are derived from cannabis plants include primary extracts prepared by such processes as for example, maceration, percolation, extraction with solvents such as C1 to C5 alcohols (e.g. ethanol), Norflurane (HFA134a), HFA227, liquid carbon dioxide under pressure and extraction using a hot gas. The primary extract may be further purified by supercritical or subcritical extraction, vaporisation and chromatography. When solvents such as those listed above are used the resultant extract may contain non-specific lipid-soluble material. This can be removed by a variety of processes including winterisation, which involves chilling to -20°C followed by filtration to remove waxy ballast, extraction with liquid carbon dioxide and by distillation.

Botanical drug substances are formulated into Botanical Drug Products which are defined in the Guidance for Industry Botanical Drug Products Draft Guidance, August 2000, US Department of Health and Human Services, Food and Drug Administration Centre for Drug Evaluation and Research as: "A botanical product that is intended for use as a drug; a drug product that is prepared from a botanical drug substance."

The one or more cannabinoids may be administered separately, sequentially or simultaneously to the non-cannabinoid anti-tumoural agent.

The one or more cannabinoids may be presented together with the non-cannabinoid chemotherapeutic agent in the form of a kit together with or without instructions to their use.

In accordance with a second aspect of the present invention there is provided a medicine comprising the cannabinoids tetrahydrocannabinol (THC) and cannabidiol (CBD) in a ratio of from 5:1 to 1:5 together with an alkylating agent.

Certain aspects of this invention are further described, by way of example only, with reference to the accompanying drawings in which:
Figure 1 is a bar chart showing the cell viability of human U87 MG astrocytoma cells after treatment with THC, CBD or a combination of THC and CBD in comparison to a control;
Figures 2a and 2b are bar charts showing in vivo cell viability data at different concentrations on two cell lines, U87MG (Fig 2a) and T98G (Fig 2b);
Figures 3a, 3b and 3c provide data suggestive of the mechanism of action of the combination for U87MG cells.
Figure 4 is a bar chart showing the cell viability of human U87 MG astrocytoma cells after treatment with an exemplary cannabinoid, THC, or a combination of an exemplary cannabinoid THC and an exemplary non-cannabinoid anti-tumour agent, tamoxifen in comparison to a control; and
Figure 5 is a bar chart showing the cell viability of human U87 MG astrocytoma cells after treatment with an exemplary cannabinoid THC, or a combination of an exemplary cannabinoid THC and an exemplary non-cannabinoid anti-tumour agent, cisplatin in comparison to a control.

### SPECIFIC DESCRIPTION

The following examples 1 to 5 illustrate how one or more cannabinoids are effective in the treatment of cancer cells both *in vitro* and *in vivo.*

Example 6 goes on to demonstrate, using a single cannabinoid for illustrative purposes, how the combination of one or more cannabinoids with a non-cannabinoid anti-tumoural agent may be better than the use of the non-cannabinoid anti-tumoural agent alone.

### Example 1: The effect of THC and CBD at inhibiting cancer cell growth in vitro.

Tetrahydrocannabinol (THC) and cannabidiol (CBD) in the form of cannabis plant extracts were dissolved in ethanol to a concentration of 100mM this was stored at -20°C until required.

Before use the cannabis plant extracts were further diluted to the desired concentration, ensuring that the concentration of ethanol was below 0.001%.

U87 human glioma cells were used throughout this experiment. The cells were maintained at 37°C in a humidified atmosphere with 5% CO₂ and 95% air.

Cells were cultured in a 75cm² culture flask in Dulbecco's Modified Eagle Medium (DMEM), which had been supplemented with 4mM L-glutamine, 100 units/ml penicillin, 100 mg/ml streptomycin, 1% sodium pyruvate, 1% non-essential amino acids and 10% heat-inactivated fetal bovine serum.

The viability of the human U87 MG astrocytoma cells were examined at various cannabinoid concentrations. The THC and CBD extracts were compared against pure THC and CBD.

### Results:

**Table 1: Cell viability of human U87 MG astrocytoma cells in culture**

| | IC50 µM (pure cannabinoids) | IC50 µM (cannabis plant extract) | IC50 µM (equivalent of pure in cannabis plant extract) |
|---|---|---|---|
| THC | 0.37 | 0.64 | 0.43 |
| CBD | 0.47 | 0.72 | 0.47 |

As can be seen from Table 1 above the THC and CBD extracts compare very favourably in activity to their corresponding pure compounds, when the amount of cannabinoid in the extract is adjusted to an equivalent amount of pure compound.

This shows that THC and CBD and their extracts are effective in inhibiting glioma cell growth.

### Example 2: The effect of a combination of THC and CBD extracts at inhibiting cancer cell growth in vitro.

This experiment tested whether a combination of THC and CBD extracts were as effective at inhibiting cell growth as the extracts alone.

The methods used were as described in Example 1 above.

### Results:

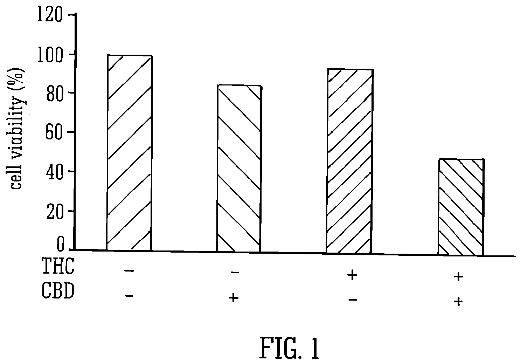

Figure 1 details a bar chart describing the cell viability of human U87 MG astrocytoma cells versus the THC and CBD extracts alone and in combination with one another.

As can be seen when the THC and CBD are used in combination the cell viability is significantly reduced in comparison to the cell viability after treatment with either THC or CBD alone.

This data suggests that the cannabinoids THC and CBD would be more effective in the treatment of tumours when used in combination.

### Example 3: The effect of a combination of THC and CBD at inhibiting cancer cell growth in vivo.

This experiment tested whether the combination of THC and CBD extracts were also effective *in vivo.*

Human U87 MG astrocytoma cells were xenografted to nude mice and the test compounds were injected peritumourally at a concentration of 15 mg/kg per day.

### Results:

**Table 2: Tumour volume relative to zero time following 15 days of treatment**

| | Tumour volume |
|---|---|
| Vehicle | 9.2 ± 0.6 |
| Pure THC | 5.1 ± 0.4 |
| THC extract | 6.6 ± 0.3 |
| THC:CBD (1:1) extract | 4.8 ± 0.3 |

As can be observed in Table 2 above the tumour volume after treatment with the 1:1 combination of THC and CBD extracts is significantly superior to the treatment with either the pure THC or the THC extract alone.

This data suggests that the cannabinoids THC and CBD would be more effective in the treatment of tumours when used in combination.

### Example 4: Effect of cannabinoid concentration on cell viability in two different cell lines.

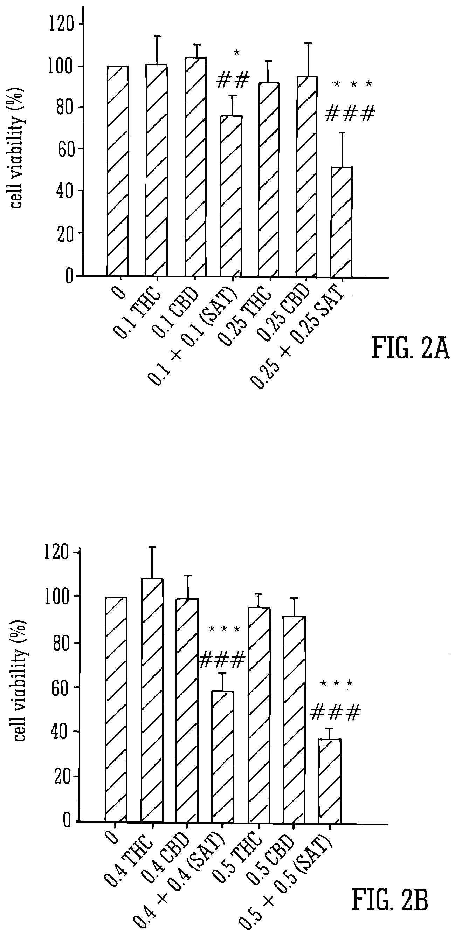

The action of THC, CBD, and a 1:1 ratio mix of THC and CBD were studied at different concentrations on two cell lines: U87MG and T98G. The cell viability data is illustrated in Figs 2a and 2b.

Referring to Fig 2a it will be seen that ineffective / sub-optimal doses of THC and CBD at 0.1ug/ml and 0.25ug/ml (greater than 90% cell viability)gave way to a statistically significant decrease in cell viability in combination (SAT), which data showed a dose dependant relationship with increased concentration (greater cytotoxicity at 0.25ug/ml).

Similar results were obtained with cell line T98G, (an alternative human glioma cell line) as is shown in Fig 2b.

### Example 5: Investigation of mechanism of action.

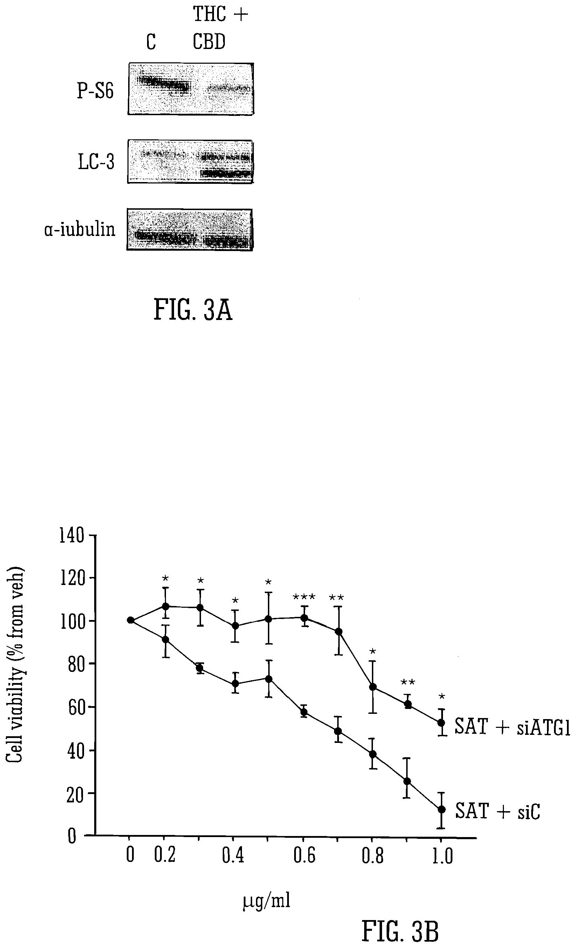

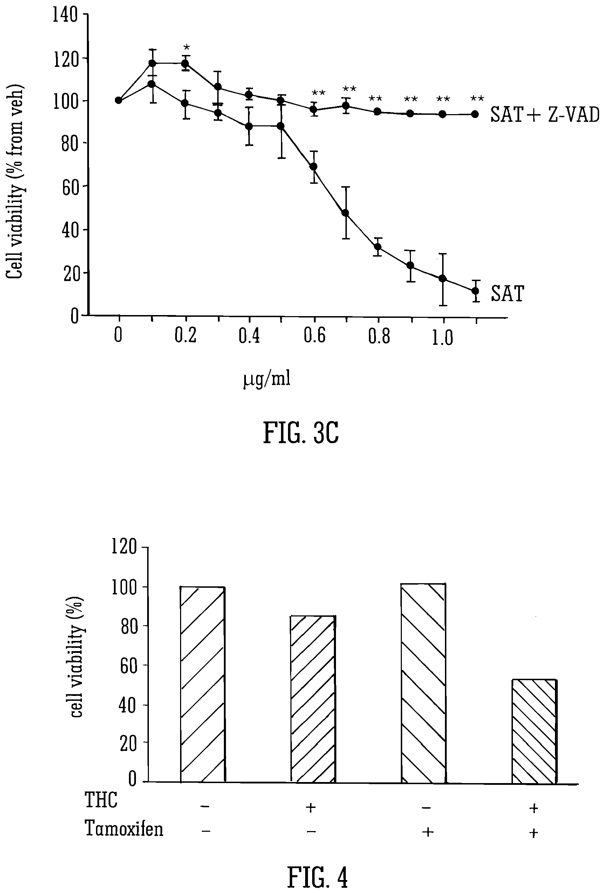

THC is known to induce cell death using a signalling route involving the gene ATG1 and pan-caspase. The results of an investigation looking at S6 phosphorylation, LC3 lipidation and the effect of an ATG1 and a pan-caspase inhibitor are shown in Figs 3a, 3b and 3c respectively.

It can be seen from Fig 3a that the THC:CBD combination (compare to control C):
- Inhibits mTORC1 activity (as determined by the levels of S6 phosphorylation);and
- Promotes accumulation of the lipidated form LC3 (a hall mark of autophagy).

Fig 3b shows that silencing the essential autophagy gene ATG1, with a selective (siATG10) siRNA inhibitor reduces induced cell death compared to cells transfected with a control siC.

Finally, Fig 3c shows that cells treated with the pancapase inhibitor Z-VAD also prevent induced cell death.

### Example 6: The effect of an exemplary cannabinoid extract in combination with exemplary non-cannabinoid anti-tumoural agents at inhibiting cancer cell growth in vitro.

This experiment tested whether a cannabinoid-containing medicine might be useful in combination with non-cannabinoid anti-tumoural agents.

Cell viability assays were performed as described in Example 1 above.

### Results:

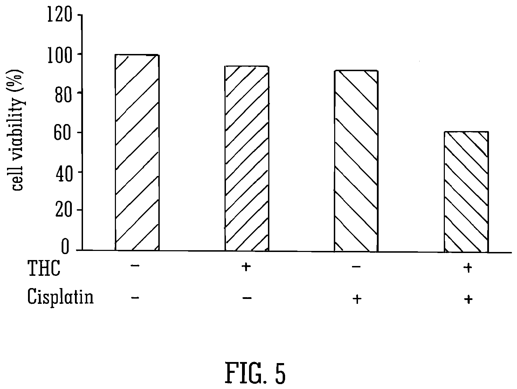

Figures 4 and 5 detail bar charts which illustrate the cell viability of human U87 MG astrocytoma cells when treated with either THC, an anti-tumoural agent or a combination of the two versus a control.

As can be seen from these figures both exemplary anti-tumoural agents that were tested; tamoxifen and cisplatin, were more efficient at reducing cell viability when used in combination with an exemplary cannabinoid, THC. It would be readily apparent from Examples 1 to 5 that similar benefits would be expected with CBD and a combination of THC and CBD.

The combination of Examples 1 to 6 demonstrate that the combination of one or more cannabinoids with a non-cannabinoid anti-tumoural drug produces a more beneficial effect than the use of the non-cannabinoid anti-tumoural drug alone.

## Claims

1. A combination of the cannabinoids tetrahydrocannabinol (THC) and cannabidiol (CBD) in a ratio of from 5:1 to 1:5 together with an alkylating agent for use in the treatment of a brain tumour wherein the THC and/ or CBD is present in an amount which would be considered sub-optimal if it were used alone.

2. A combination of the cannabinoids tetrahydrocannabinol (THC) and cannabidiol (CBD) for use as claimed in claim 1 wherein the THC is present in an amount which would be considered sub-optimal if it were used alone.

3. A combination of the cannabinoids tetrahydrocannabinol (THC) and cannabidiol (CBD) for use as claimed in claim 1 wherein the CBD is present in an amount which would be considered sub-optimal if it were used alone.

4. A combination of the cannabinoids tetrahydrocannabinol (THC) and cannabidiol (CBD) for use as claimed in claim 1 wherein the THC and CBD are present in an amount which would be considered sub-optimal if they were used alone.

5. A combination of the cannabinoids tetrahydrocannabinol (THC) and cannabidiol (CBD) for use as claimed in any of the preceding claims, wherein the treatment of the brain tumour is to reduce cell viability, inhibit cell growth or reduce tumour volume.

6. A combination of the cannabinoids tetrahydrocannabinol (THC) and cannabidiol (CBD) for use as claimed in any of the preceding claims, wherein the THC and CBD are in the ratio of from between 2:1 to 1:2.

7. A combination of the cannabinoids tetrahydrocannabinol (THC) and cannabidiol (CBD) for use as claimed in any of the preceding claims, wherein the THC and CBD are in the ratio of approximately 1:1.

8. A combination of the cannabinoids tetrahydrocannabinol (THC) and cannabidiol (CBD) for use as claimed in any of the preceding claims, wherein the total cannabinoid content is in the range of between 5 and 100mg.

9. A combination of the cannabinoids tetrahydrocannabinol (THC) and cannabidiol (CBD) for use as claimed in claim 1, wherein the alkylating agent is selected from the group consisting of: alkyl sulfonates; busulfan; ethyleneimines and methylmelamines; hexamethymelamine; altretamine; thiotepa; nitrogen mustards; cyclophosphamide; mechlorethamine; mustine; uramustine; uracil mustard; melphalan; chlorambucil; ifosfamide; nitrosureas; carmustine; cisplatin; streptozocin; triazenes; decarbazine; imidazotetrazines; and temozolomide.

10. A combination of the cannabinoids tetrahydrocannabinol (THC) and cannabidiol (CBD) for use as claimed in claim 9, wherein the alkylating agent is one of: cisplatin; temozolomide; and carmustine.

11. A combination of the cannabinoids tetrahydrocannabinol (THC) and cannabidiol (CBD) for use as claimed in any of the preceding claims, wherein the brain tumour is a glioma tumour.

12. A combination of the cannabinoids tetrahydrocannabinol (THC) and cannabidiol (CBD) for use as claimed in claim 11, wherein the brain tumour is a glioblastoma multiforme (GBM).

13. A combination of the cannabinoids tetrahydrocannabinol (THC) and cannabidiol (CBD) for use as claimed in any of the preceding claims, wherein the THC and CBD are present as plant extracts, as pure compounds, or a combination of the two.

14. A combination of the cannabinoids tetrahydrocannabinol (THC) and cannabidiol (CBD) for use as claimed in claim 13, wherein the plant extract is in the form of a botanical drug substance.

15. A combination of the cannabinoids tetrahydrocannabinol (THC) and cannabidiol (CBD) for use as claimed in any of the preceding claims, wherein the THC and CBD are administered separately, sequentially or simultaneously to the non-cannabinoid anti-tumoural agent.

16. A medicine comprising the cannabinoids tetrahydrocannabinol (THC) and cannabidiol (CBD) in a ratio of from 5:1 to 1:5 together with an alkylating agent, wherein the THC and/or CBD is present in an amount which would be considered sub-optimal if it were used alone.

## Patentansprüche

1. Kombination aus den Cannabinoiden Tetrahydrocannabinol (THC) und Cannabidiol (CBD) in einem Verhältnis von 5:1 bis 1:5 zusammen mit einem Alkylierungsmittel zur Verwendung bei der Behandlung eines Hirntumors, wobei das THC und/oder das CBD in einer Menge vorliegen, die als suboptimal gelten würde, wenn sie alleine eingesetzt würde.

2. Kombination aus den Cannabinoiden Tetrahydrocannabinol (THC) und Cannabidiol (CBD) zur Verwendung nach Anspruch 1, wobei das THC in einer Menge vorliegt, die als suboptimal gelten würde, wenn sie alleine eingesetzt würde.

3. Kombination aus den Cannabinoiden Tetrahydrocannabinol (THC) und Cannabidiol (CBD) zur Verwendung nach Anspruch 1, wobei das CBD in einer Menge vorliegt, die als suboptimal gelten würde, wenn sie alleine eingesetzt würde.

4. Kombination aus den Cannabinoiden Tetrahydrocannabinol (THC) und Cannabidiol (CBD) zur Verwendung nach Anspruch 1, wobei das THC und das CBD in einer Menge vorliegen, die als suboptimal gelten würde, wenn sie alleine eingesetzt würde.

5. Kombination aus den Cannabinoiden Tetrahydrocannabinol (THC) und Cannabidiol (CBD) zur Verwendung nach einem der vorhergehenden Ansprüche, wobei die Behandlung des Hirntumors dazu dient, Zellviabilität zu reduzieren, Zellwachstum zu hemmen oder Tumorvolumen zu reduzieren.

6. Kombination aus den Cannabinoiden Tetrahydrocannabinol (THC) und Cannabidiol (CBD) zur Verwendung nach einem der vorhergehenden Ansprüche, wobei das THC und das CBD in dem Verhältnis von zwischen 2:1 bis 1:2 sind.

7. Kombination aus den Cannabinoiden Tetrahydrocannabinol (THC) und Cannabidiol (CBD) zur Verwendung nach einem der vorhergehenden Ansprüche, wobei das THC und das CBD in dem Verhältnis von annähernd 1:1 sind.

8. Kombination aus den Cannabinoiden Tetrahydrocannabinol (THC) und Cannabidiol (CBD) zur Verwendung nach einem der vorhergehenden Ansprüche, wobei der Gesamtgehalt von Cannabinoiden in dem Bereich von zwischen 5 und 100 mg ist.

9. Kombination aus den Cannabinoiden Tetrahydrocannabinol (THC) und Cannabidiol (CBD) zur Verwendung nach Anspruch 1, wobei das Alkylierungsmittel ausgewählt ist aus der Gruppe bestehend aus: Alkylsulfonaten; Busulfan; Ethyleniminen und Methylmelaminen; Hexamethymelamin; Altretamin; Thiotepa; Stickstofflosten; Cyclophosphamid; Mechlorethamin; Mustin; Uramustin; Uracillost; Melphalan; Chlorambucil; Ifosfamid; Nitrosoharnstoffen; Carmustin; Cisplatin; Streptozocin; Triazenen; Decarbazin; Imidazotetrazinen; und Temozolomid.

10. Kombination aus den Cannabinoiden Tetrahydrocannabinol (THC) und Cannabidiol (CBD) zur Verwendung nach Anspruch 9, wobei das Alkylierungsmittel eines der Folgenden ist: Cisplatin; Temozolomid; und Carmustin.

11. Kombination aus den Cannabinoiden Tetrahydrocannabinol (THC) und Cannabidiol (CBD) zur Verwendung nach einem der vorhergehenden Ansprüche, wobei der Hirntumor ein Gliomtumor ist.

12. Kombination aus den Cannabinoiden Tetrahydrocannabinol (THC) und Cannabidiol (CBD) zur Verwendung nach Anspruch 11, wobei der Hirntumor ein Glioblastoma multiforme (GBM) ist.

13. Kombination aus den Cannabinoiden Tetrahydrocannabinol (THC) und Cannabidiol (CBD) zur Verwendung nach einem der vorhergehenden Ansprüche, wobei das THC und das CBD als Pflanzenextrakte, als reine Verbindungen oder als eine Kombination aus den beiden vorliegen.

14. Kombination aus den Cannabinoiden Tetrahydrocannabinol (THC) und Cannabidiol (CBD) zur Verwendung nach Anspruch 13, wobei der Pflanzenextrakt in der Form einer botanischen Arzneimittelsubstanz ist.

15. Kombination aus den Cannabinoiden Tetrahydrocannabinol (THC) und Cannabidiol (CBD) zur Verwendung nach einem der vorhergehenden Ansprüche, wobei das THC und das CBD getrennt, aufeinanderfolgend oder gleichzeitig zu dem nicht-cannabinoiden antitumorösen Wirkstoff verabreicht werden.

16. Arzneimittel, umfassend die Cannabinoide Tetrahydrocannabinol (THC) und Cannabidiol (CBD) in einem Verhältnis von 5:1 bis 1:5 zusammen mit einem Alkylierungsmittel, wobei das THC und/oder das CBD in einer Menge vorliegen, die als suboptimal gelten würde, wenn sie alleine eingesetzt würde.

## Revendications

1. Combinaison des cannabinoïdes tétrahydrocannabinol (THC) et cannabidiol (CBD) dans un rapport de 5:1 à 1:5 avec un agent d'alkylation pour une utilisation dans le traitement d'une tumeur cérébrale, ledit THC et/ou ledit CBD étant présents en une quantité qui serait considérée comme étant sous-optimale s'il était utilisé seul.

2. Combinaison des cannabinoïdes tétrahydrocannabinol (THC) et cannabidiol (CBD) pour une utilisation selon la revendication 1, ledit THC étant présent en une quantité qui serait considérée comme étant sous-optimale s'il était utilisé seul.

3. Combinaison des cannabinoïdes tétrahydrocannabinol (THC) et cannabidiol (CBD) pour une utilisation selon la revendication 1, ledit CBD étant présent en une quantité qui serait considérée comme étant sous-optimale s'il était utilisé seul.

4. Combinaison des cannabinoïdes tétrahydrocannabinol (THC) et cannabidiol (CBD) pour une utilisation selon la revendication 1, ledit THC et ledit CBD étant présents en une quantité qui serait considérée comme étant sous-optimale s'ils étaient utilisés seuls.

5. Combinaison des cannabinoïdes tétrahydrocannabinol (THC) et cannabidiol (CBD) pour une utilisation selon l'une quelconque des revendications précédentes, ledit traitement de la tumeur cérébrale consistant à réduire la viabilité des cellules, inhiber la croissance des cellules ou réduire le volume de la tumeur.

6. Combinaison des cannabinoïdes tétrahydrocannabinol (THC) et cannabidiol (CBD) pour une utilisation selon l'une quelconque des revendications précédentes, ledit THC et ledit CBD étant dans un rapport compris entre 2:1 et 1:2.

7. Combinaison des cannabinoïdes tétrahydrocannabinol (THC) et cannabidiol (CBD) pour une utilisation selon l'une quelconque des revendications précédentes, ledit THC et ledit CBD étant dans un rapport d'environ 1:1.

8. Combinaison des cannabinoïdes tétrahydrocannabinol (THC) et cannabidiol (CBD) pour une utilisation selon l'une quelconque des revendications précédentes, la teneur totale en cannabinoïdes étant comprise entre 5 et 100 mg.

9. Combinaison des cannabinoïdes tétrahydrocannabinol (THC) et cannabidiol (CBD) pour une utilisation selon la revendication 1, ledit agent d'alkylation étant choisi dans le groupe constitué par : les sulfonates d'alkyle ; le busulfan ; les éthylèneimines et les méthylmélamines ; l'hexaméthymélamine ; l'altrétamine ; le thiotépa ; les moutardes à l'azote; le cyclophosphamide ; la méchloréthamine ; la mustine ; l'uramustine ; la moutarde à l'uracile ; le melphalan ; le chlorambucil ; l'ifosfamide ; les nitrosourées ; la carmustine ; le cisplatine ; la streptozocine ; les triazènes ; la décarbazine ; les imidazotétrazines ; et le témozolomide.

10. Combinaison des cannabinoïdes tétrahydrocannabinol (THC) et cannabidiol (CBD) pour une utilisation selon la revendication 9, ledit agent d'alkylation étant l'un parmi : le cisplatine ; le témozolomide ; et la carmustine.

11. Combinaison des cannabinoïdes tétrahydrocannabinol (THC) et cannabidiol (CBD) pour une utilisation selon l'une quelconque des revendications précédentes, ladite tumeur cérébrale étant une tumeur gliome.

12. Combinaison des cannabinoïdes tétrahydrocannabinol (THC) et cannabidiol (CBD) pour une utilisation selon la revendication 11, ladite tumeur cérébrale étant un glioblastome multiforme (GBM).

13. Combinaison des cannabinoïdes tétrahydrocannabinol (THC) et cannabidiol (CBD) pour une utilisation selon l'une quelconque des revendications précédentes, ledit THC et ledit CBD étant présents sous la forme d'extraits de plantes, sous la forme de composés purs ou une combinaison des deux.

14. Combinaison des cannabinoïdes tétrahydrocannabinol (THC) et cannabidiol (CBD) pour une utilisation selon la revendication 13, ledit extrait végétal étant sous la forme d'une substance médicamenteuse botanique.

15. Combinaison des cannabinoïdes tétrahydrocannabinol (THC) et cannabidiol (CBD) pour une utilisation selon l'une quelconque des revendications précédentes, ledit THC et ledit CBD étant administrés séparément, séquentiellement ou simultanément à l'agent antitumoral non cannabinoïde.

16. Médicament comprenant les cannabinoïdes tétrahydrocannabinol (THC) et cannabidiol (CBD) dans un rapport de 5:1 à 1:5 avec un agent d'alkylation, ledit THC et/ou ledit CBD étant présents en une quantité qui serait considérée comme étant sous-optimale s'il était utilisé seul.
